# EUROPÄISCHE PATENTANMELDUNG

(11) **EP 0 782 985 A1**
(43) Veröffentlichungstag der Anmeldung: **09.07.1997**
(21) Anmeldenummer: 96120746.1
(22) Anmeldetag: 23.12.1996
(51) Int. Cl.: C07D 203/02, C07D 203/08

(54) **Verfahren zur Herstellung von Aziridinen**

(30) Priorität: 04.01.1996 DE 19600156
(71) Anmelder: BASF AKTIENGESELLSCHAFT, 67056 Ludwigshafen (DE)
(72) Erfinder: Grosch, Georg Heinrich, Dr., 67098 Bad Dürkheim (DE); Gehrer, Eugen, Dr., 67069 Ludwigshafen (DE); Steuerle, Ulrich, Dr., 69124 Heidelberg (DE); König, Cornelia, 69198 Schriesheim (DE); Eder, Michael, 67069 Ludwigshafen (DE)

(57) **Zusammenfassung**

Verfahren zur Herstellung von Aziridinen der allgemeinen Formel I 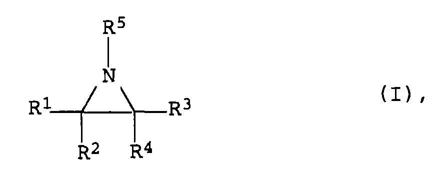 in der
- R¹,R²,R³,R⁴: Wasserstoff, C₁- bis C₈-Alkyl, C₃- bis C₈-Cycloalkyl oder Aryl und
- R⁵: Wasserstoff, C₁- bis C₈-Alkyl, C₃- bis C₈-Cycloalkyl, Aryl, Benzyl, C₁- bis C₈-Hydroxyalkyl oder C₁- bis C₈-Aminoalkyl bedeuten,
durch Umsetzung von Alkanolaminen der allgemeinen Formel II 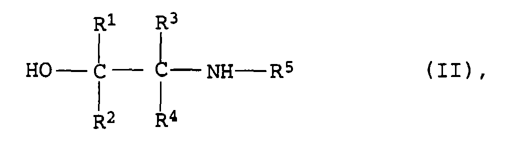 in der die Substituenten R¹, R², R³, R⁴ und R⁵ die oben genannten Bedeutungen haben, in der Gasphase bei Temperaturen von 200 bis 600°C und einem Druck von 0,001 bis 5 bar an Heterogenkatalysatoren, indem man als Heterogenkatalysatoren mit dreiwertigen Metallionen dotierte Alkali-, Erdalkaliphosphate oder deren Gemische einsetzt sowie neue Katalysatoren.

## Beschreibung

Die vorliegende Erfindung betrifft ein Verfahren zur Herstellung von Aziridinen durch Umsetzung von Alkanolaminen an phosphathaltige Träger- oder Vollkontakt-Katalysatoren in der Gasphase.

Die Herstellung von Aziridinen, insbesondere Ethylenimin, durch Dehydratisierung von Alkanolaminen in der Gasphase an Molekularsieben aus Aluminiumsilikaten, Aluminiumphosphaten oder Silicium-Aluminiumphosphaten ist aus der WO-A-89/05797 bekannt.

Aus US-A-4 289 656, US-A-4 301 036, US-A-4 337 175, US-A-4 358 405, US-A-376 732 und US-A-4 477 591 sind Katalysatoren auf Basis Niob/Tantaloxid mit Zusatz von Erdalkalimetalloxiden und/oder Eisen/Chromoxiden zur katalytischen Gasphasendehydratisierung von Monoethanolamin zu Ethylenimin bekannt.

Weitere Katalysatoren zur intramolekularen Wasserabspaltung aus Alkanolaminen in der Gasphase sind Oxidmassen, die Silicium oder Phosphor als wesentliche Bestandteile enthalten. Katalysatoren dieser Art sind beispielsweise aus EP-A-227 461, EP-A-228 898 und EP-A-230 776 bekannt.

Setzt man bei der Aziridinherstellung die in den oben genannten Literaturstellen beschriebenen Katalysatoren ein, so ergeben sich einige Fakten, die bei der Umsetzung in die technische Realität prohibitiv sind.

Bei den zuvor genannten Stand der Technik mit nicht phosphorhaltigen Katalysatoren sind die Ausbeuten und Selektivitäten in der Regel zu gering, um die Aziridinherstellung wirtschaftlich betreiben zu können. Bei den phosphorhaltigen Katalysatoren sind die Ausbeuten und Selektivitäten zwar hoch genug, jedoch lassen hier die Standzeiten des Katalysators zu wünschen übrig. Die aus EP-A-228 898 und EP-A-230 776 bekannten Katalysatoren zeigen zwar reproduzierbar hohe Selektivitäten und Ausbeuten, jedoch lassen sich die angegebenen Standzeiten wegen des Phosphoraustrages aus den Katalysatoren nicht reproduzieren.

Die bekannten Katalysatoren haben weiterhin den Nachteil, daß in unerwünschtem Maße Oligomere und Polymere des Aziridins entstehen.

Der vorliegenden Erfindung lag daher die Aufgabe zugrunde, den zuvor genannten Nachteilen abzuhelfen.

Demgemäß wurde ein neues und verbessertes Verfahren zur Herstellung von Aziridinen der allgemeinen Formel I 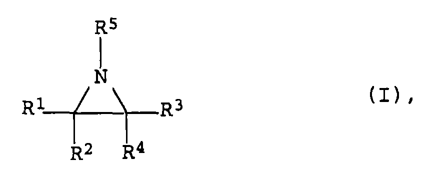 in der
- R¹,R²,R³,R⁴: Wasserstoff, C₁- bis C₈-Alkyl, C₃- bis C₈-Cycloalkyl oder Aryl und
- R⁵: Wasserstoff, C₁- bis C₈-Alkyl, C₃- bis C₈-Cycloalkyl, Aryl, Benzyl, C₁- bis C₈-Hydroxyalkyl oder C₁- bis C₈-Aminoalkyl bedeuten,
durch Umsetzung von Alkanolaminen der allgemeinen Formel II 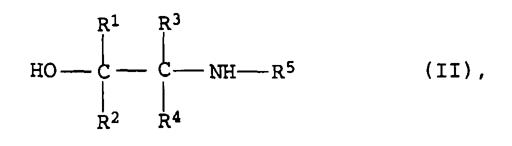 in der die Substituenten R¹, R², R³, R⁴ und R⁵ die oben genannten Bedeutungen haben, in der Gasphase bei Temperaturen von 200 bis 600°C und einem Druck von 0,001 bis 5 bar an Heterogenkatalysatoren gefunden, welches dadurch gekennzeichnet ist, daß man als Heterogenkatalysatoren mit dreiwertigen Metallionen dotierte Alkali-, Erdalkaliphosphate oder deren Gemische einsetzt sowie neue Katalysatoren.

Das erfindungsgemäße Verfahren läßt sich wie folgt durchführen:

Man kann die Alkanolamine II bei Temperaturen von 200 bis 600°C, bevorzugt 300 bis 550°C, besonders bevorzugt 350 bis 500°C und einem Druck von 0,001 bis 5 bar, bevorzugt 0,01 bis 2 bar, besonders bevorzugt 0,1 bis 1 bar in der Gasphase an einem Heterogenkatalysator diskontinuierlich oder bevorzugt kontinuierlich, in der Gasphasen-Fahrweise umsetzen (dehydratisieren).

Als Heterogenkatalysatoren eignen sich beispielsweise Trägerkatalysatoren oder Vollkontaktkatalysatoren von Alkali-, Erdalkaliphosphaten oder deren Gemischen wie Lithium-, Natrium-, Kalium-, Rubidium-, Cäsium-, Beryllium-, Magnesium-, Calcium-, Strontium- und Bariumphosphate, bevorzugt Natrium-, Kalium-, Rubidium-, Cäsium-, Calcium-, Strontium- und Bariumphosphate, besonders bevorzugt Cäsium- und Bariumphosphate, die mit dreiwertigen Metallionen dotiert sind.

Als inerte Träger eignen sich beispielsweise Siliciumdioxid, Aluminiumoxid, Siliciumdioxid/Aluminiumoxid, Titandioxid, Zirkondioxid, Tone, Alumosilikate oder siliciumhaltige nichtporöse Gläser, bevorzugt Siliciumoxid, Titandioxid, Zirkondioxid oder siliciumhaltige nichtporöse Gläser, besonders bevorzugt Siliciumoxid oder siliciumhaltige nichtporöse Gläser.

Das Aufbringen der Aktivmasse auf einen inerten Träger kann durch Imprägnieren, Tränken, Aufrotieren, Gasphasenabscheidungen oder andere allgemein bekannte Techniken erfolgen.

Der erfindungsgemäße Katalysator enthält in seiner Aktivmasse (Alkali-, Erdalkaliphosphaten oder deren Gemischen) 0,01 bis 20 Gew.-%, bevorzugt 0,05 bis 10 Gew.-%, besonders bevorzugt 0,5 bis 5 Gew.-% (bezogen auf die Menge der Aktivmasse) eines oder mehrerer dreiwertiger Metallionen der Lanthaniden, der Actiniden, der Gruppen IIIb, IVb, Vb, VIb, VIIb, VIII, IIIa, Va des Periodensystems der Elemente oder deren Gemische wie Scandium, Yttrium Lanthan, Titan, Zirkon, Hafnium, Vanadin, Niob, Tantal, Chrom, Molybdän, Wolfram, Mangan, Technetium Rhenium, Eisen, Cobalt, Nickel, Ruthenium, Rhodium, Palladium, Osmium, Iridium, Platin, Bor, Aluminium, Gallium, Indium, Thallium, Phosphor, Arsen, Antimon, Wismut, Cer, Praseodym, Neodym, Promethium, Samarium, Europium, Thorium, Protactinium und Uran. Bevorzugt sind dreiwertige Metallionen der Gruppen IIIb, IVb, Vb, VIb, VIIb, VIII, IIIa des Periodensystems der Elemente oder deren Gemische wie Scandium, Yttrium Lanthan, Titan, Zirkon, Hafnium, Vanadin, Niob, Tantal, Chrom, Molybdän, Wolfram, Mangan, Technetium Rhenium, Eisen, Cobalt, Nickel, Ruthenium, Rhodium, Palladium, Osmium, Iridium, Platin, Bor, Aluminium, Gallium, Indium und Thallium, besonders bevorzugt Eisen, Aluminium, Chrom, Bor, Mangan, Cobalt, Europium und Vanadium, insbesondere Eisen, Aluminium, Chrom und Bor.

Die Isolierung der entstandenen Aziridine I aus dem Reaktionsgemisch kann beispielsweise mit Hilfe einer wäßrig-alkalischen Absorbtionsflüssigkeiten und nachgeschalteten Destillation erfolgen. Die wäßrig-alkalischen Absorptionsflüssigkeiten können mehrfach verwendet werden. Geeignete Flüssigkeiten dieser Art sind beispielsweise wäßrige NaOH oder KOH-Lösungen.

Die Substituenten R¹, R², R³, R⁴ und R⁵ in den Verbindungen I und II haben folgende Bedeutungen:
- R¹ R² R³ R⁴, R⁵: - Wasserstoff,
- C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl, bevorzugt C₁- bis C₄-Alkyl wie Methyl, Ethyl, n-Propyl, iso-Propyl, n-Butyl, iso-Butyl, sec.-Butyl und tert.-Butyl, besonders bevorzugt Methyl und Ethyl,
- C₃- bis C₈-Cycloalkyl wie Cyclopropyl, Cyclopentyl, Cyclohexyl, Cycloheptyl und Cyclooctyl, bevorzugt Cyclopentyl, Cyclohexyl und Cyclooctyl, besonders bevorzugt Cyclopentyl und Cyclohexyl,
- Aryl wie Phenyl, 1-Naphthyl und 2-Naphthyl, bevorzugt Phenyl,
- R⁵: - zusätzlich
- Benzyl,
- C₁- bis C₈-Hydroxyalkyl, bevorzugt C₁- bis C₄-Hydroxyalkyl, wie Hydroxymethyl, 1-Hydroxyethyl, 2-Hydroxyethyl, 1-Hydroxy-n-propyl, 2-Hydroxy-n-propyl und 3-Hydroxy-n-propyl, besonders bevorzugt C₁- bis C₂-Hydroxyalkyl wie Hydroxymethyl, 1-Hydroxyethyl und 2-Hydroxyethyl,
- C₁- bis C₈-Aminoalkyl, bevorzugt C₁- bis C₄-Aminoalkyl wie Aminomethyl, 1-Aminoethyl, 2-Aminoethyl, 1-Amino-n-propyl, 2-Amino-n-propyl und 3-Amino-n-propyl, besonders bevorzugt C₁- bis C₂-Aminoalkyl wie Aminomethyl, 1-Aminoethyl und 2-Aminoethyl.

### Beispiele

### Katalysator A

300 g Siliperl AF 125 (Fa. Engelhardt) wurden nach Vortrocknung mit einer Lösung aus 9,21 g ortho-Phosphorsäure in 400 ml Wasser getränkt, bei 120°C/35 mbar einrotiert, bei 120°C eine Stunde getrocknet und bei 500° 5h kalziniert. Der erhaltene Träger wurde mit einer Lösung aus 9,51 g Cäsiumacetat und 7,41 g Bariumacetat in 400 ml Wasser getränkt, bei 120°C/35 mbar einrotiert, bei 120°C eine Stunde getrocknet, bei 500°C 5h vorkalziniert und bei 1100°C 1h endkalziniert.

### Katalysator B

24,2 g Siliperl AF 125 (Fa. Engelhardt) wurden nach Vortrocknung mit einer Lösung aus 0,74 g ortho-Phosphorsäure in 20 ml Wasser getränkt, bei 120°C/35 mbar einrotiert, bei 120°C eine Stunde getrocknet und bei 500°C 5h kalziniert. Der erhaltene Träger wurde mit einer Lösung aus 0,75 g Cäsiumacetat, 0,58 g Bariumacetat und 0,067 g Aluminiumnitrat-Nonahydrat in 20 ml Wasser getränkt, bei 120°C/35mbar einrotiert, bei 120°C eine Stunde getrocknet, bei 500°C 3h vorkalziniert und bei 1100°C 1h endkalziniert.

### Katalysator C

29 g Siliperl AF 125 (Fa. Engelhardt) wurden nach Vortrocknung mit einer Lösung aus 0,89 g ortho-Phosphorsäure in 20 ml Wasser getränkt, bei 120°C/35mbar einrotiert, bei 120°C eine Stunde getrocknet und bei 500°C 5h kalziniert. Der erhaltene Träger wurde mit einer Lösung aus 0,90 g Cäsiumacetat, 0,7 g Bariumacetat und 0,059 g Eisen-(III)-chlorid in 20 ml Wasser getränkt, bei 120°C/35 mbar einrotiert, bei 120°C eine Stunde getrocknet, bei 500°C 3h vorkalziniert und bei 1100°C 1h endkalziniert.

### Katalysator D

Der Träger hergestellt wie bei Katalysator C wurde mit einer Lösung aus 0,85 g Cäsiumacetat, 0,66 g Bariumacetat und 0,12 g Eisen-(III)-chlorid in 20 ml Wasser getränkt und wie bei Katalysator C beschrieben getrocknet, vor- und endkalziniert.

### Katalysator E

Der Träger hergestellt wie bei Katalysator C wurde mit einer Lösung aus 0,8 g Cäsiumacetat, 0,62 g Bariumacetat und 0,178 g Eisen-(III)-chlorid in 20 ml Wasser getränkt und wie bei Katalysator C beschrieben getrocknet, vor- und endkalziniert.

### Katalysator F

Der Träger hergestellt wie bei Katalysator B wurde mit einer Lösung aus 0,7 g Cäsiumacetat, 0,55 g Bariumacetat und 0,13 g Aluminiumnitrat-Nonahydrat in 20 ml Wasser getränkt und wie bei Katalysator B beschrieben getrocknet, vor- und endkalziniert.

### Katalysator G

Der Träger hergestellt wie bei Katalysator B wurde mit einer Lösung aus 0,66 g Cäsiumacetat, 0,51 g Bariumacetat und 0,2 g Aluminiumnitrat-Nonahydrat in 20 ml Wasser getränkt und wie bei Katalysator B beschrieben getrocknet, vor- und endkalziniert.

### Katalysator H

29,1 g Siliperl AF 125 (Fa. Engelhardt) wurden nach Vortrocknung mit einer Lösung aus 0,9 g ortho-Phosphorsäure in 20 ml Wasser getränkt, bei 120°C/35mbar einrotiert, bei 120°C eine Stunde getrocknet und bei 500°C 5h kalziniert. Der erhaltene Träger wurde mit einer Lösung aus 0,8 g Cäsiumacetat, 0,62 g Bariumacetat und 0,004 g Borsäure in 20 ml Wasser getränkt und wie bei Katalysator C beschrieben getrocknet, vor- und endkalziniert.

### Katalysator J

Der Träger hergestellt wie bei Katalysator H wurde mit einer Lösung aus 0,8 g Cäsiumacetat, 0,62 g Bariumacetat und 0,067 g Scandiumsulfat-Hexahydrat in 20 ml Wasser getränkt und wie bei Katalysator C beschrieben getrocknet, vor- und endkalziniert.

### Katalysator K

Der Träger hergestellt wie bei Katalysator H wurde mit einer Lösung aus 0,8 g Cäsiumacetat, 0,62 g Bariumacetat und 0,037 g Chrom-(III)-chlorid-Hexahydrat in 20 ml Wasser getränkt und wie bei Katalysator C beschrieben getrocknet, vor- und endkalziniert.

### Katalysator L

Der Träger hergestellt wie bei Katalysator H wurde mit einer Lösung aus 0,8 g Cäsiumacetat, 0,62 g Bariumacetat und 0,093 g Galliumnitrat-Monohydrat in 20 ml Wasser getränkt und wie bei Katalysator C beschrieben getrocknet, vor- und endkalziniert.

### Katalysator M

Der Träger hergestellt wie bei Katalysator H wurde mit einer Lösung aus 0,8 g Cäsiumacetat, 0,62 g Bariumacetat und 0,093 g Indiumnitrat-Pentahydrat in 20 ml Wasser getränkt und wie bei Katalysator C beschrieben getrocknet, vor- und endkalziniert.

### Herstellung von Aziridin

25 g Katalystor wurden in einen kontinuierlich betriebenen, elektrisch beheizten Linearreaktor (Innendurchmesser 30 mm; Länge 300 mm) mit Innentemperaturmessung gefüllt. Der Zulauf des Eduktgemisches erfolgte von oben. Es wurden 120 Liter/h Stickstoff und 30 g/h Ethanolamin dosiert. Das Ethanolamin enthielt bis zu 1 Gew.-% Wasser. Das restliche Reaktorvolumen wurde je nach Bedarf mit Inertmaterial (Glaskugeln, Porzellanringe, etc.) befüllt. Die Versuchstemperatur betrug 400°C und die Versuchsdauer 12 Stunden.

Die Austräge wurden gaschromatographisch getrennt nach Flüssig- und Gasphase untersucht. Die Flüssigphase wurde mit NaOH stabilisiert. Als interner Standard wurde Butylmethylether eingesetzt.

Man erhielt die in Tabelle 1 angegebenen Ergebnisse:

**Tabelle 1**

| Gasphasendehydratisierung von Ethanolamin zu Ethylenimin | | | |
|---|---|---|---|
| Katalysator | Umsatz [%] | Selektivität [%] | Ausbeute [%] |
| A | 87,6 | 69,4 | 60,8 |
| B | 87,1 | 68,9 | 60,0 |
| C | 91,8 | 57,5 | 52,8 |
| D | 84,8 | 37,3 | 31,7 |
| E | 80,2 | 39,7 | 31,8 |
| F | 86,4 | 34,9 | 30,1 |
| G | 87,2 | 38,7 | 33,7 |
| H | 74,6 | 70,3 | 52,4 |
| J | 78,2 | 47,9 | 37,4 |
| K | 81,3 | 76,6 | 62,2 |
| L | 88,4 | 15,4 | 13,6 |
| M | 88,1 | 15,3 | 13,5 |

Die Katalysatorproben wurden vor und nach der Reaktion durch Elementaranalyse untersucht. Die Ergebnisse sind in Tabelle 2 zusammengestellt.

**Tabelle 2**

| Katalysator | M³⁺ [%] vorher | M³⁺ [%] nachher | Phosphor [%] vorher | Phosphor [%] nachher |
|---|---|---|---|---|
| A | 0,85 M³⁺=Ba | 0,85 M³⁺=Ba | 0,77 | 0,70 |
| B | 0,14 | 0,14 | 0,78 | 0,76 |
| C | 0,08 | 0,07 | 0,84 | 0,74 |
| D | 0,11 | 0,12 | 0,83 | 0,84 |
| E | 0,15 | 0,16 | 0,80 | 0,78 |
| F | 0,17 | 0,16 | 0,75 | 0,72 |
| G | 0,17 | 0,17 | 0,75 | 0,75 |
| H | 0,03 | 0,02 | 0,83 | 0,79 |
| J | 0,09 | 0,06 | 0,82 | 0,79 |
| K | 0,02 | 0,03 | 0,84 | 0,81 |
| L | 0,06 | 0,08 | 0,91 | 0,88 |
| M | 0,02 | 0,03 | 0,94 | 0,89 |

## Patentansprüche

1. Verfahren zur Herstellung von Aziridinen der allgemeinen Formel I 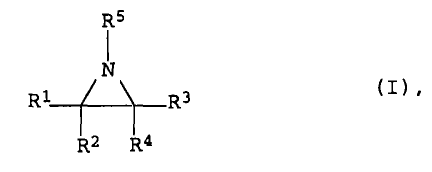 in der
R¹,R²,R³,R⁴ Wasserstoff, C₁- bis C₈-Alkyl, C₃- bis C₈-Cycloalkyl oder Aryl und
R⁵ Wasserstoff, C₁- bis C₈-Alkyl, C₃- bis C₈-Cycloalkyl, Aryl, Benzyl, C₁- bis C₈-Hydroxyalkyl oder C₁- bis C₈-Aminoalkyl bedeuten,
durch Umsetzung von Alkanolaminen der allgemeinen Formel II 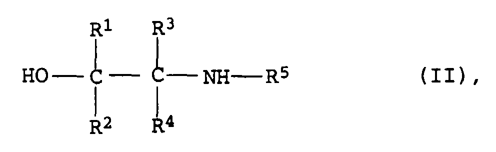 in der die Substituenten R¹, R², R³, R⁴ und R⁵ die oben genannten Bedeutungen haben, in der Gasphase bei Temperaturen von 200 bis 600°C und einem Druck von 0,001 bis 5 bar an Heterogenkatalysatoren, dadurch gekennzeichnet, daß man als Heterogenkatalysatoren mit dreiwertigen Metallionen dotierte Alkali-, Erdalkaliphosphate oder deren Gemische einsetzt.

2. Verfahren zur Herstellung von Aziridinen nach Anspruch 1, dadurch gekennzeichnet, daß man als Alkali-, Erdalkaliphosphate Cäsium-, Bariumphosphate oder deren Gemische einsetzt.

3. Verfahren zur Herstellung von Aziridinen nach Anspruch 1, dadurch gekennzeichnet, daß man als Heterogenkatalysatoren Träger- oder Vollkontakt-Katalysatoren einsetzt.

4. Verfahren zur Herstellung von Aziridinen nach Anspruch 1, dadurch gekennzeichnet, daß der Gehalt an dreiwertigem Metall zwischen 0,01 und 20 Gew.-% bezogen auf die Aktivmasse beträgt.

5. Verfahren zur Herstellung von Aziridinen nach Anspruch 1, dadurch gekennzeichnet, daß man als dreiwertige Metallionen der Lanthaniden, der Actiniden, der Gruppen IIIb, IVb, Vb, VIb, VIIb, VIIIb, IIIa, Va des Periodensystems der Elemente oder deren Gemische einsetzt.

6. Verfahren zur Herstellung von Aziridinen nach Anspruch 1, dadurch gekennzeichnet, daß man als dreiwertige Metallionen Eisen, Aluminium, Chrom, Bor, Mangan, Vanadium, Europium, Cobalt oder deren Gemische einsetzt.

7. Verfahren zur Herstellung von Aziridinen nach Anspruch 1, dadurch gekennzeichnet, daß man als dreiwertige Metallionen Eisen, Aluminium, Chrom, Bor oder deren Gemische einsetzt.

8. Verfahren zur Herstellung von Aziridinen nach Anspruch 1, dadurch gekennzeichnet, daß die Substituenten R¹, R², R³, R⁴ und R⁵ gleichzeitig Wasserstoff bedeuten.

9. Verfahren zur Herstellung von Aziridinen nach Anspruch 1, dadurch gekennzeichnet, daß man die Umsetzung in der Wirbelschicht bei einem Druck von 0,001 bis 2 bar durchführt.

10. Verfahren zur Herstellung von Aziridinen nach den Ansprüchen 1 bis 2, dadurch gekennzeichnet, daß man die Umsetzung unter einem Druck von 0,01 bis 1 bar durchführt.

11. Katalysator bestehend aus 99,9 bis 80 Gew.-% Alkali-, Erdalkaliphosphate oder deren Gemische und 0,01 und 20 Gew.-% dreiwertige Metallionen der Lanthaniden, der Actiniden, der Gruppen IIIb, IVb, Vb, VIb, VIIb, VIIIb, IIIa, Va des Periodensystems der Elemente oder deren Gemische gegebenenfalls auf einem inerten Träger.
